# EUROPÄISCHE PATENTSCHRIFT

(11) **EP 2 293 027 B1**
(45) Veröffentlichungstag und Bekanntmachung des Hinweises auf die Patenterteilung: **08.08.2012**
(21) Anmeldenummer: 10008458.1
(22) Anmeldetag: 13.08.2010
(51) Int. Cl.: G01F 15/06, F24D 17/00, G01K 3/00

(54) **Verfahren zur Abschätzung des Gefährdungspotentials einer Verkeimung mit einem Durchflusszähler**
Method for rating the hazard potential of a microbial contamination with a flow meter
Procédé d'estimation du danger potentiel d'une contamination microbienne à l'aide d'un débitmètre

(30) Priorität: 04.09.2009 DE 102009040261
(43) Veröffentlichungstag der Anmeldung: 09.03.2011
(73) Patentinhaber: Hydrometer GmbH, 91522 Ansbach (DE)
(72) Erfinder: Gaugler, Ulrich, 91746 Weidenbach (DE); Stefke, Frank, 97258 Gollhofen (DE)
(74) Vertreter: Diehl Patentabteilung

(56) Entgegenhaltungen:
- DE-A1-102007 009 007
- Jürg Schmid: Wasser-Probenahme für Legionellenuntersuchungen - Info-Blatt Nr. B04/2, 14 July 2008 (2008-07-14), XP55007158, [retrieved on 2011-09-14]

## Beschreibung

Die Erfindung betrifft ein Verfahren zur Überwachung des Gefährdungspotentials bezüglich einer Verkeimung an einem Durchflusszähler sowie einen zur Durchführung des Verfahrens ausgebildeten Durchflusszähler.

Ein bekanntes Problem in der Trinkwasserversorgung ist die Aufrechterhaltung einer hinreichenden Wasserqualität, insbesondere hinsichtlich der Gefahr der Verkelmung. Es ist bekannt, dass die Wahrscheinlichkeit für eine Verkeimung des Wassers mit zunehmender Temperatur und Verweilzeit des Wassers in der Leitung ansteigt. Allerdings ist es bislang nur schwer möglich, das Gefährdungspotential tatsächlich einauschätzen und bei hoher Gefährdung entsprechende Gegenmaßnahmen einzuleiten.

Aus der DE 10 2007 009 007 A1 ist eine in einem Wasserhahn angeordnete Vorrichtung zur Überwachung der Spülaktivitäten einer Wasserleitung bekannt. Dort werden die Messwerte eines Durchflusssensors und eines Temperatursensors kontinuierlich in einen Messwertspelcher geschrieben und mit hinterlegten Vorgabewerten abgeglichen. Das Ergebnis dieses Abgleichs wird über ein Ausgabemodul angezeigt, wobei eine Überschreitung der hinterlegten Grenzwerte durch den Wechsel von einer grünen LED-Anzeige zu einer gelben (innerhalb eines Toleranzbereichs) oder roten LED-Anzeige (außerhalb des Toleranzbereiches) signalisiert wird. Diese recht einfache Mess- und Auswertemethode ist jedoch wenig verlässlich.

Der Erfindung liegt daher die Aufgabe zugrunde, ein Verfahren zur verlässlichen Überwachung der Verkeimungsgefahr zu schaffen, welches an einem Durchflusszähler einsetzbar und einfach realisierbar ist.

Zur Lösung dieses Problems Ist ein Verfahren zur Überwachung des Gefährdungspotentials bezüglich einer Verkeimung an einem Durchflusszähler mit den Merkmalen der Patentansprüche 1 oder 5 vorgesehen.

Es wird folglich eine einfach zu realisierende Erweiterung eines Durchflusszählers vorgeschlagen, indem wenigstens ein weiteres Register einer Speichereinrichtung verwendet wird, um darin temperaturabhängig eine physikalische Größe und/oder eine davon abgeleitete Größe temperaturabhängig zu speichern. Bei der Größe handelt es sich mithin um einen kumulativ wachsenden Wert, der sich insbesondere auf die Wassermenge bzw. die Verweilzeit des Wassers in dem sich an den Durchflusszähler anschließenden Leitungssystem bezieht. Dabei bieten sich insbesondere, wie im Folgenden auch noch näher diskutiert wird, das durch den Durchflusszähler ohnehin gemessene und in einem Register gespeicherte gezapfte Volumen und/oder die Zeit ohne Zapfvorgang, in der das Wasser also still steht, an. Beispielsweise kann also vorgesehen sein, die Gesamtmenge an gezapftem Wasser in einem Register vorzuhalten und ein weiteres Register vorzusehen, in dem insbesondere nur die Menge an Wasser oberhalb einer bestimmten Temperaturschwellwerts eingespeichert wird, worauf im Folgenden noch näher eingegangen werden wird.

Wie bereits erwähnt, sind die Standzeit des Wassers im Leitungssystem und auch die Menge gezapften Wassers in einem bestimmten Temperaturbereich grundsätzlich sinnvolle Auswahlmöglichkeiten für die im erfindungsgemäßen Verfahren zu betrachtende Größe, wobei beide einzeln, aber auch gleichzeitig betrachtet werden können.

Zunächst sei der Fall näher diskutiert, in dem als physikalische Größe das Volumen betrachtet wird. Durchflusszähler für Wasser zählen meist grundsätzlich das abgezapfte Volumen. Dieses wird in einem Register gespeichert. Erfindungsgemäß wird nun ein weiteres Register vorgesehen, in das temperaturabhängig das Volumen oder eine davon abgeleitete Größe eingespeichert werden kann, um ein Maß für die Wasserqualität zu erhalten. Beispielsweise kann vorgesehen sein, dass grundsätzlich das gezapfte Volumen temperaturunabhängig gespeichert wird und in einem weiteren Register das gezapfte Volumen oberhalb eines Temperaturschwellwerts für die Wassertemperatur abgelegt wird.

Dabei ist allgemein vorgesehen, dass bei jedem Zapfvorgang ein vorbestimmtes Volumen gezapft sein muss, ehe ein Speichern im Register erfolgt. Auf diese Weise wird sichergestellt, dass durch den Temperaturfühler auch tatsächlich die Wassertemperatur aufgenommen wird und nicht die Raumtemperatur des Raumes, in dem sich der Durchflusszähler befindet. Dem Temperaturfühler wird mithin Zeit gegeben, auch tatsächlich die Temperatur des letztlich gezapften Wassers zu messen. Beispielsweise kann vorgesehen sein, dass das vorbestimmte Volumen einen Liter beträgt. Es sei angemerkt, dass es rein grundsätzlich auch denkbar ist, bei jedem Zapfvorgang eine vorbestimmte Zeit, beispielsweise einige Sekunden, zu warten, ehe ein Speichern in dem Register erfolgt. So kann ein ähnlicher Effekt erzielt werden, jedoch gibt das durchgeflossene Volumen einen besseren Anhalt.

Erfindungsgemäß ist weiterhin vorgesehen, dass eine von dem Volumen und der Wassertemperatur abgeleitete Größe in das Register gespeichert wird, insbesondere das Produkt des Volumens und des Temperaturunterschieds von der gemessenen Wassertemperatur zu dem Temperaturschwellwert. Auf diese Weise wird immer auch die konkret vorhandene Temperatur berücksichtigt, so dass beispielsweise einen Temperaturschwellwert nur sehr knapp überschreitende Wassertemperaturen zu einer eher geringen Erhöhung des Wertes im Register führen, während hohe Temperaturen einen entsprechend großen Zuwachs erzielen, insbesondere, da eine höhere Temperatur meist auch ein höheres Verkeimungspotential darstellt.

In vorteilhafter weiterer Ausgestaltung kann bei Betrachtung des Volumens als physikalische Größe vorgesehen sein, dass das dem Volumen zugeordnete Register zyklisch gelöscht wird oder der darin gespeicherte Wert zyklisch um einen vorbestimmten Wert erniedrigt wird. Auf diese Weise wird ein Zeitbezug erst gegeben. Wird nämlich beispielsweise ein Alarmschwellwert betrachtet, so würde dieser nach einer sehr langen Zeit auch bei nur kurzfristig erhöhten Temperaturen erreicht werden, ohne dass gegebenenfalls tatsächlich eine aktuelle Verschlechterung der Wasserqualität vorliegt. Folglich kann regelmäßig das Register gelöscht werden, oder aber bevorzugt der im Register gespeicherte Wert zyklisch um einen vorbestimmten Wert erniedrigt werden. Auf diese Weise kann der Wert im Register als ein Volumenfortschritt pro Zeit verstanden werden. Der vorbestimmte Wert, der zyklisch abgezogen wird, ist letztlich ein Erfahrungswert, der für entsprechende folgende Installationen bzw. Leitungssysteme bestimmt werden kann. Selbstverständlich kann der Wert in einem Register niemals unter 0 sinken.

Wird das Volumen als solches im Register gespeichert, so kann vorgesehen sein, dass als Maß der Quotient aus dem im Register gespeicherten Volumen und dem insgesamt durchgeflossenen Volumen gebildet wird. Es wird also das Verhältnis zwischen dem Stand des Registers zur Überwachung und dem Gesamtvolumenstand betrachtet, so dass festgestellt werden kann, welcher Anteil des durchgeflossenen Wassers beispielsweise eine zu hohe Temperatur hat. Hierfür kann dann beispielsweise ein Alarmschwellwert festgelegt werden, wenn ein zu großer Anteil des Wassers eine zu große Temperatur aufweist.

Alternativ wird als physikalische Größe die Zeit bei stehendem Zähler betrachtet, also immer dann, wenn kein Wasser durch den Durchflusszähler fließt. Sie ist also ein Maß dafür, wie lange Wasser einer zu hohen Temperatur in dem sich an den Durchflusszähler anschließenden Leitungssystem verweilt. Konkret kann also vorgesehen sein, dass die Zeit, während der die Wassertemperatur einen Temperaturschwellwert überschreitet, in einem Register zur Überwachung der Wasserqualität aufaddiert wird.

Dabei kann mit besonderem Vorteil vorgesehen sein, dass das der Zeit zugeordnete Register gelöscht wird, wenn die Wassertemperatur während eines Abzapfvorgangs einen vorbestimmten Wert, insbesondere den Temperaturschwellwert, unterschreitet. Denn sobald wieder kühles Wasser in das Leitungssystem gelangt, ist die Standzeit des warmen Wassers darin beendet. Dieser Fall tritt insbesondere über Nacht auf, wenn lange kein Wasser gezapft wird. Hier bietet sich eine Qualitätsüberwachung im Übrigen besonders an, da so die Qualität des Wassers, welches beispielsweise für das morgendliche Zähneputzen verwendet wird, überwacht werden kann.

Weiterhin kann vorgesehen sein, dass bei einer Temperatur unterhalb des dem Register zugeordneten unteren Temperaturschwellwertes der im Register abgespeicherte Wert abhängig von der Zeit erniedrigt wird. Dabei ist der Wert, um den pro Zeiteinheit erniedrigt wird, niedriger als der Wert, um den pro Zeiteinheit bei Überschreitung des Temperaturschwellwerts erhöht würde. Somit wird letztlich beobachtet, ob die Temperatur eine zu lange Zeit auf einem zu hohen Wert verweilt.

Es kann weiterhin vorgesehen sein, dass als Maß der Quotient aus der im Register gespeicherten Zeit und einer Gesamtzeit gebildet wird. Die Gesamtzeit kann dabei die Gesamtbetriebszeit oder die Gesamtstillstandszeit sein, insbesondere seit dem letzten Löschen des Registers. Auch in diesem Fall kann beispielsweise ein Alarmschwellwert vorgesehen sein, wenn das Wasser einen zu großen Anteil der Zeit eine zu große Temperatur hatte.

Allgemein sind selbstverständlich bezüglich der Zeit wie auch bezüglich des Volumens oder allgemein der physikalischen Größe noch eine Vielzahl sinnvoller anderer Auswertungsrnöglichkeiten denkbar. Insbesondere können auch komplexere Auswertungen erfolgen, wenn Daten über die Wasserqualität regelmäßig beispielsweise an den Versorger gesendet werden.

Aus der temperaturabhängig in dem Register abgespeicherten Größe kann nun ein Maß für die Wasserqualität, insbesondere die Verkeimungsgefahr, ermittelt werden. Insbesondere kann das Maß unmittelbar die im Register abgespeicherte Größe sein, so dass zur Ermittlung des Maßes nur das Register ausgelesen werden muss. Schließlich wird das Maß für die Wasserqualität ausgewertet, wobei in vorteilhafter weiterer Ausgestaltung der Erfindung vorgesehen sein kann, dass bei Überschreitung eines Alarmschwellwerts für das Maß ein Alarmsignal ausgegeben wird. Ist also beispielsweise zuviel warmes Wasser in das Leitungssystem gelangt oder stand zu lange warmes Wasser in dem Leitungssystem, so kann der Benutzer und/oder der Versorger über einen Alarm hierauf hingewiesen werden. Selbstverständlich ist es auch denkbar, das Maß oder eine davon abgeleitete Information über ein Anzeigemittel darzustellen und jederzeit abrufbar zu gestalten.

So ist es im Rahmen der vorliegenden Erfindung möglich, die Wasserqualität, insbesondere das Gefährdungspotential bezüglich einer Verkeimung, ständig zu überwachen, wobei letztlich lediglich wenigstens ein weiteres Register, also wenigstens ein weiterer Speicherplatz, benötigt werden. Bestehende Durchflusszähler sind somit einfach nachrüstbar, insbesondere, wenn ein Temperaturfühler bereits vorgesehen ist.

In weiterer Ausgestaltung der vorliegenden Erfindung kann vorgesehen sein, dass das Maß oder eine davon abgeleitete Information, insbesondere das Alarmsignal, drahtlos, insbesondere über Funk, an eine weitere Einrichtung übertragen wird. Moderne Durchflusszähler sind über Kommunikationskanäle auslesbar. Derartige Kommunikationsverbindungen können im Rahmen des erfindungsgemäßen Verfahrens benutzt werden, um das Maß selber oder eine daraus abgeleitete Information über die Wasserqualität, insbesondere das Verkeimungspotential, zu übertragen. Die weitere Einrichtung kann dabei eine Nebenstelle oder dergleichen sein, über die gegebenenfalls auch ein Alarm über andere Kommunikationswege weiterverbreitet werden kann, beispielsweise per SMS.

Mehrere einer physikalischen Größe zugeordnete Register können für verschiedene Temperaturschwellwerte und/oder verschiedene Temperaturintervalle vorgesehen werden. So ist eine bessere Aufschlüsselung nach verschiedenen Temperaturbereichen auch im Nachhinein noch denkbar, was in der Auswertung benutzt werden kann, beispielsweise über eine entsprechende Wichtung oder dergleichen.

In besonders vorteilhafter Ausgestaltung der vorliegenden Erfindung kann bei der Ermittlung der von der physikalischen Größe abgeleiteten Größe der zeitliche Verlauf der Wassertemperatur, insbesondere das Differential des Temperaturverlaufs, berücksichtigt werden. Auf diese Weise können externe Effekte, die den zeitlichen Verlauf der Wassertemperatur des Zulaufs beeinflussen können, bei der Ermittlung des Maßes und gegebenenfalls auch bei dessen Auswertung berücksichtigt werden. Verläuft beispielsweise eine Wasserleitung unmittelbar neben einer Fernwärmezuführung, kann es vorkommen, dass die Wassertemperatur zunächst kurzfristig etwas absinkt, wenn kühleres Wasser nachläuft, danach jedoch wieder ansteigt, da das neben der Fernwärmeleitung stehende Wasser den Durchflusszähler erreicht. Derartiges kann im Rahmen der vorliegenden Erfindung vorteilhaft ebenso berücksichtigt werden.

Neben dem Verfahren betrifft die vorliegende Erfindung auch einen Durchflusszähler für Wasser, umfassend eine zur Durchführung des Verfahrens nach einem der vorangehenden Ansprüche ausgebildete Recheneinrichtung und eine Speichereinrichtung.

Der Durchflusszähler umfasst also einen Temperaturfühler, über den entweder unmittelbar oder indirekt die Wassertemperatur gemessen werden kann, und ist zudem zur Erfassung wenigstens einer weiteren physikalischen Größe, insbesondere des durchgeflossenen Volumens und/oder der Zeit bei stehendem Wasser, ausgebildet. Die Recheneinrichtung, die mit der Speichereinrichtung in Kommunikationsverbindung steht, ist dazu ausgebildet, in Abhängigkeit von der Wassertemperatur und dem Betriebszustand des Zählers die physikalische Größe und/oder eine daraus abgeleitete Größe in einem speziellen Register zu speichern, um hieraus ein Maß für die Wasserqualität zu ermitteln und dieses auszuwerten. Die Auswertung kann selbstverständlich auch wenigstens teilweise an einem entfernten Ort, beispielsweise einer weiteren Einrichtung erfolgen. Die bezüglich des erfindungsgemäßen Verfahrens getätigten Ausführungen gelten analog.

Vorzugsweise kann der Durchflusszähler weiterhin eine Kommunikationseinrichtung zur insbesondere drahtlosen Kommunikation, insbesondere über Funk, mit einer weiteren Einrichtung, beispielsweise einer Nebenstelle, umfassen, welche dann zur Ausgabe einer aus dem Maß abgeleiteten Information, insbesondere eines Alarms, und/oder zur Weiterleitung des Maßes und/oder einer daraus abgeleiteten Information über andere Kommunikationswege ausgebildet sein kann. So kann beispielsweise über ein visuelles und/oder akustisches Ausgabemittel ein Alarm erzeugt werden, wenn ein Qualitätsschwellwert überschritten wird. Selbstverständlich ist es auch denkbar, solcherlei Ausgabemittel direkt am Durchflusszähler vorzusehen.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Prinzipskizze eines erfindungsgemäßen Ourchflusszählers, und
- Fig. 2: einen Ablaufplan eines Ausführungsbeispiels des erfindungsgemäßen Verfahrens.

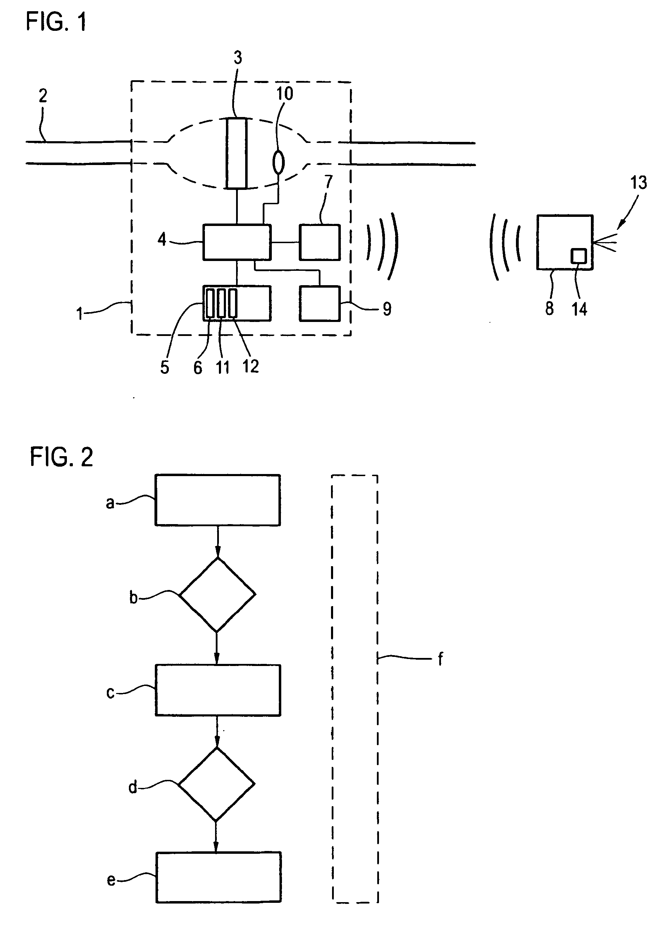

Fig. 1 zeigt die Prinzipskizze der wichtigsten Komponenten eines erfindungsgemäßen Durchflusszählers 1. Er umfasst ein in eine Wasserleitung 2 eingekoppeltes Messwerk 3, über das das durchfließende Volumen bestimmt werden kann. Das Volumen wird durch eine Recheneinrichtung 4 bestimmt und als Gesamtvolumen in einem Register 6 in einer Speichereinrichtung 5 abgespeichert.

Der Durchflusszähler 1 umfasst ferner eine Kommunikationseinrichtung 7, über die durch Funk Daten, beispielsweise der Gesamtvolumenstand im Register 6, an eine weitere Einrichtung 8, beispielsweise eine Nebenstelle, übertragen werden können.

Über ein Ausgabemittel 9, hier ein optisches Ausgabemittel, können jedoch auch am Durchflusszähler 1 Informationen, beispielsweise über den Gesamtvolumenstand, abgelesen werden.

Ferner umfasst der Durchflusszähler 1 einen Temperaturfühler 10, über den die Wassertemperatur gemessen werden kann. Auch diese Daten werden an die Recheneinrichtung 4 kommuniziert. Diese ist nun zur Durchführung des erfindungsgemäßen Verfahrens ausgebildet.

Dazu umfasst die Speichereinrichtung 5 im hier dargestellten Beispiel zwei weitere Register 11, 12. In das Register 11 wird immer dann, wenn Wasser durchfließt, dessen Wassertemperatur einen Temperaturschwellwert überschreitet, das entsprechende Volumen eingespeichert. Es erfolgt folglich eine temperaturabhängige Speicherung des Volumens: Das Register 11 gibt an, wie viel Wasser, das wärmer als der Temperaturschwellwert ist, den Durchflusszähler 1 passiert, während im Register 6 das Gesamtvolumen gespeichert wird. Denkbar ist im Übrigen auch, dass der Wert im Register 11 erst erhöht wird, wenn ein vorbestimmtes Volumen gezapft wurde.

Aus den in den Registern 6 und 11 gespeicherten Werten kann die Steuereinrichtung 4 ein Maß für die Wasserqualität ermitteln, vorliegend der Quotient aus dem im Register 11 gespeicherten Volumen und dem im Register 6 gespeicherten Volumen. Dieses Maß wird ständig überwacht, insbesondere dahingehend, ob es einen vorgegebenen Alarmschwellwert überschreitet, der aus Erfahrungswerten hergeleitet wurde. Wird der Alarmschwellwert überschritten, ist von einem hohen Gefährdungspotential durch Verkeimung auszugehen, da ein zu großer Anteil des durchgeflossenen Wassers eine zu hohe Temperatur hatte. Dann kann entsprechend ein Alarm auf dem Ausgabemittel 9, welches hierzu auch akustische Komponenten umfassen kann, ausgegeben werden, oder es wird ein Alarmsignal über die Kommunikationseinrichtung 7 an die weitere Einrichtung 8 übertragen, welche diesen über weitere Kommunikationswege 13 oder dort angeordnete Ausgabemittel 14 ausgeben und so zur Kenntnis bringen kann. So kann eine Information des Verwenders des Wassers genauso erreicht werden wie des Wasserversorgers.

Es sei im Übrigen angemerkt, dass grundsätzlich auch eine kontinuierliche, zyklische oder auf Anfrage erfolgende Datenübermittlung bezüglich des Maßes für die Wasserqualität erfolgen kann, insbesondere kann beim Versorger oder der weiteren Einrichtung 8 auch eine weitere Auswertung erfolgen.

In das Register 11 kann im Übrigen auch eine vom Volumen abgeleitete Größe temperaturabhängig abgespeichert werden, insbesondere eine von dem Volumen und der Wassertemperatur abgeleitete Größe, beispielsweise das Produkt des Volumens und des Temperaturunterschieds von der gemessenen Wassertemperatur zu dem Temperaturschwellwert. So werden verschiedene Volumina je nach ihrer Wassertemperatur unterschiedlich gewichtet. Denkbar sind jedoch auch kompliziertere arithmetische Abhängigkeiten, in die auch weitere Größen eingehen können, beispielsweise kann der zeitliche Verlauf der Wassertemperatur, insbesondere das Differential des Temperaturverlaufs, berücksichtigt werden.

Durch die Recheneinrichtung 4 wird des Weiteren in zyklischen Abständen der im Register 11 gespeicherte Wert erniedrigt, wobei er 0 nicht unterschreiten kann. Auf diese Weise entspricht der im Register 6 gespeicherte Wert im Wesentlichen einen Volumenfortschritt, wobei der abzuziehende Wert beispielsweise unter Berücksichtigung üblicher Abzapfmengen ermittelt werden kann.

In dem Register 12 wird eine weitere grundsätzlich zunehmende physikalische Größe temperaturabhängig gespeichert, nämlich die Zeit, in der kein Durchfluss vorliegt. Liegt kein Durchfluss vor, und ist die Wassertemperatur größer als ein Temperaturschwellwert, insbesondere derselbe Temperaturschwellwert, der schon bezüglich des Registers 11 berücksichtigt wird, so wird die Zeit, für die diese hohe Temperatur vorliegt, in das Register 12 gespeichert. Gleichzeitig kann vorgesehen sein, dass auch die gesamte Zeit, in der kein Durchfluss vorliegt, in der Speichereinrichtung 5 abgelegt wird, beispielsweise in einem hier nicht dargestellten Register. Dann kann als weiteres Maß für die Wasserqualität der Quotient der im Register 12 abgespeicherten Zeit und der Gesamtzeit analog ermittelt und ausgewertet werden. Denn auch die Standzeit des Wassers ist relevant für das Verkeimungspotential.

Der Inhalt des Registers 12 wird durch die Recheneinrichtung 4 in zweierlei Hinsicht zusätzlich beeinflusst. Zum einen wird das der Zeit zugeordnete Register 12 gelöscht, wenn die durch den Temperaturfühler 10 gemessene Wassertemperatur während eines Abzapfvorgangs den Temperaturschwellwert unterschreitet. Dann ist die Standzeit des zu warmen Wassers beendet.

Gleichzeitig wird jedoch der im Register 12 abgespeicherte Wert immer dann proportional zu der vergangenen Zeit erniedrigt, wenn die gemessene Wassertemperatur unterhalb des Temperaturschwellwertes liegt, wobei die Erniedrigung geringer als die Erhöhung ist, folglich der Wert, um den pro Zeiteinheit erniedrigt wird, niedriger ist als der Wert, um den pro Zeiteinheit bei Überschreitung des Temperaturschwellwerts erhöht wird.

Fig. 2 zeigt in Form eines Ablaufplans einige grundsätzliche Schritte des erfindungsgemäßen Verfahrens, vorliegend bezüglich des Volumens als zu betrachtende physikalische Größe. Immer dann, wenn Wasser abgezapft wird, wird das durchfließende Volumen und die Temperatur bestimmt, Schritt a. Das durchfließende Volumen wird grundsätzlich in das Register 6 der Speichereinrichtung 5 eingespeichert. Dann wird aber überprüft, Schritt b, ob die Wassertemperatur einen Temperaturschwellwert überschreitet. Trifft dies nicht zu, wird für die Zeit des Abzapfvorgangs weiter gemessen. Ist die Temperatur jedoch höher als der Temperaturschwellwert, wird das Volumen in einem Schritt c auch dem Register 11 hinzugefügt, welches folglich temperaturabhängig geführt wird. In einem Schritt d wird als Maß für die Wasserqualität der Quotient aus dem im Register 11 temperaturabhängig gespeicherten Volumen und dem im Register 6 gespeicherten Gesamtvolumen gebildet und überprüft, ob dieses einen Alarmschwellwert überschreitet. Falls nein, wird bei Schritt a fortgefahren. Falls ja, wird in einem Schritt e auf eine der oben bereits diskutierten Methoden ein Alarmsignal gegeben.

Selbstverständlich sind, wie oben bereits erwähnt, auch andere Auswertungsmethoden zusätzlich oder alternativ denkbar, genau wie in das Register 11 ein von dem Volumen abgeleiteter Wert gespeichert werden kann, beispielsweise von der Differenz der Wassertemperatur zum Schwellwert und dem Volumen.

Analog zu dem in Fig. 2 gezeigten Vorgehen wird im Durchflusszähler 1 selbstverständlich parallel die im Register 12 gespeicherte Zeit überwacht und ausgewertet. Es sei an dieser Stelle noch angemerkt, dass auch eine kombinierte Auswertung der beiden Register 11 und 12 zu einem Maß denkbar ist.

Ein seitlich nur angedeuteter Schritt f weist darauf hin, dass der Wert im Register 11 zyklisch um einen geringen Volumenwert erniedrigt wird, vgl. die hierzu bereits getätigten Ausführungen.

## Patentansprüche

1. Verfahren zur Überwachung des Gefährdungspotentials bezüglich einer Verkeimung an einem Durchftusszähter(1), wobei
- abhängig von einer durch einen Temperaturfühler (10) ermittelten Wassertemperatur bei Überschreitung wenigstens eines Temperaturschwellwerts für die Wassertemperatur wenigstens eine von dem über den Durchflusszähler (1) erfassten Volumen und der Wassertemperatur abgeleitete Größe in wenigstens einem Register (11) einer Speichereinrichtung (5) abgespeichert wird,
- aus der wenigstens einen im Register (11) gespeicherten Größe ein Maß für die Verkeimungsgefahr ermittelt und ausgewertet wird und
- bei jedem Zapfvorgang ein vorbestimmtes Volumen gezapft sein muss, ehe ein Speichern in dem Register erfolgt.

2. Verfahren nach Anspruch 1, **dadurch gekennzeichnet, dass** das dem Volumen zugeordnete Register (11) zyklisch gelöscht wird oder der darin gespeicherte Wert zyklisch um einen vorbestimmten Wert erniedrigt wird.

3. Verfahren nach Anspruch 1 oder 2, **dadurch gekennzeichnet**, dassals abgeleitet Größe , das Produkt des Volumens und des Temperaturunterschieds von der gemessenen Wassertemperatur zu dem Temperaturschwellwert in das Register (11) gespeichert wird.

4. Verfahren nach Anspruch 1 oder 2, **dadurch gekennzeichnet, dass** als Maß der Quotient aus dem im Register (11) gespeicherten Volumen und dem insgesamt durchgeflossenen Volumen gebildet wird.

5. Verfahren zur Überwachung des Gefährdungspotentials bezüglich einer Verkeimung an einem Durchftusszähter(1), wobei
- abhängig von einer durch einen Temperaturfühler (10) ermittelten Wassertemperatur bei Überschreitung wenigstens eines Temperaturschwellwerts für die Wassertemperatur wenigstens die über den Durchflusszähler (1) erfasste Zeit bei stehendem Zähler und/oder eine davon abgeleitete Größe in wenigstens einem Register (12) einer Speichereinrichtung (5) abgespeichert wird und
- aus der wenigstens einen im Register (12) gespeicherten Größe ein Maß für die Verkeimungsgefahr ermittelt und ausgewertet wird.

6. Verfahren nach Anspruch 5, **dadurch gekennzeichnet, dass** das der Zeit zugeordnete Register (12) gelöscht wird, wenn die Wassertemperatur während eines Abzapfvorgangs einen vorbestimmten Wert, insbesondere den Temperaturschwellwert, unterschreitet.

7. Verfahren nach Anspruch 5 oder 6, **dadurch gekennzeichnet, dass** bei einer Temperatur unterhalb des dem Register (12) zugeordneten unteren Temperaturschwellwertes der im Register (12) abgespeicherte Wert abhängig von der Zeit erniedrigt wird.

8. Verfahren nach einem der Ansprüche 5 bis 7, **dadurch gekennzeichnet, dass** als Maß der Quotient aus der im Register (12) gespeicherten Zeit und einer Gesamtzeit gebildet wird.

9. Verfahren nach einem der vorangehenden Ansprüche, **dadurch gekennzeichnet, dass** bei Überschreitung eines Alarmschwellwerts für das Maß ein Alarmsignal ausgegeben wird.

10. Verfahren nach einem der vorangehenden Ansprüche, **dadurch gekennzeichnet, dass** das Maß oder eine davon abgeleitete Information, insbesondere das Alarmsignal, drahtlos, insbesondere über Funk, an eine weitere Einrichtung (8) ubertragen wird.

11. Verfahren nach einem der vorangehenden Ansprüche, **dadurch gekennzeichnet, dass** mehrere einer physikalischen Größe zugeordnete Register für verschiedene Temperaturschwellwerte und/oder verschiedene Temperaturintervalle vorgesehen sind.

12. Verfahren nach einem der vorangehenden Ansprüche, **dadurch gekennzeichnet, dass** bei der Ermittlung der von der physikalischen Größe abgeleiteten Größe der zeitliche Verlauf der Wassertemperatur, insbesondere das Differenzial des Temperaturverlaufs, berücksichtigt wird.

13. Durchflusszähler (1) für Wasser, umfassend eine zur Durchführung des Verfahrens nach einem der vorangehenden Ansprüche ausgebildete Recheneinrichtung (4) und eine Speichereinrichtung (5).

## Claims

1. Method for monitoring the potential danger with respect to build-up of germs at a flow meter (1), wherein
- depending on a water temperature ascertained by a temperature sensor (10), at least one parameter, which is derived from the volume captured via the flow meter (1) and from the water temperature, is stored in at least one register (11) of a storage device (5) when at least one temperature threshold value for the water temperature is exceeded,
- from the at least one parameter stored in the register (11) a measure for the risk of build-up of germs is ascertained and evaluated and
- at each removal operation, a predetermined volume must be removed before storage in the register is effected.

2. Method according to Claim 1, **characterized in that** the register (11) associated with the volume is cleared cyclically or the value stored therein is reduced cyclically by a predetermined value.

3. Method according to Claim 1 or 2, **characterized in that** the derived parameter stored in the register (11) is the product of the volume and of the temperature difference between the measured water temperature and the temperature threshold value.

4. Method according to Claim 1 or 2, **characterized in that**, as the measure, the quotient of the volume stored in the register (11) and the total flowed-through volume is formed.

5. Method for monitoring the potential danger with respect to build-up of germs at a flow meter (1), wherein
- depending on a water temperature ascertained by a temperature sensor (10), at least the time for an inactive meter captured via the flow meter (1) and/or a parameter which is derived therefrom is stored in at least one register (12) of a storage device (5) when at least one temperature threshold value for the water temperature is exceeded, and
- from the at least one parameter stored in the register (12) a measure for the risk of build-up of germs is ascertained and evaluated.

6. Method according to Claim 5, **characterized in that** the register (12) associated with the time is cleared when the water temperature during a removal operation falls below a predetermined value, in particular the temperature threshold value.

7. Method according to Claim 5 or 6, **characterized in that**, if a temperature is below the lower temperature threshold value associated with the register (12), the value stored in the register (12) is reduced in dependence on the time.

8. Method according to one of Claims 5 to 7, **characterized in that**, as the measure, the quotient of the time stored in the register (12) and a total time is formed.

9. Method according to one of the preceding claims, **characterized in that** an alarm signal is output for the measure if an alarm threshold value is exceeded.

10. Method according to one of the preceding claims, **characterized in that** the measure or information derived therefrom, in particular the alarm signal, is transferred to a further device (8) wirelessly, in particular by radio.

11. Method according to one of the preceding claims, **characterized in that** a plurality of registers which are associated with a physical parameter for different temperature threshold values and/or different temperature intervals are provided.

12. Method according to one of the preceding claims, **characterized in that**, in the ascertainment of the parameter derived from the physical parameter, the time profile of the water temperature, in particular the differential of the temperature profile, is taken into consideration.

13. Flow meter (1) for water, comprising a computation device (4), which is configured for carrying out the method according to one of the preceding claims, and a storage device (5).

## Revendications

1. Procédé de surveillance du potentiel de risque concernant une contamination microbienne au niveau d'un compteur débitmètre (1), selon lequel
- suivant une température de l'eau déterminée par une sonde de température (10) et en cas de dépassement d'au moins une valeur de seuil de température pour la température de l'eau, au moins une grandeur, dérivée du volume détecté par le biais du compteur débitmètre (1) et de la température de l'eau, est enregistrée dans au moins un registre (11) d'un dispositif de mémorisation (5),
- un indice pour le risque de contamination microbienne est déterminé à partir de l'au moins une grandeur enregistrée dans le registre (11) et interprété et
- un volume prédéfini doit être soutiré à chaque opération de soutirage avant qu'un enregistrement dans le registre n'ait lieu.

2. Procédé selon la revendication 1, **caractérisé en ce que** le registre (11) associé au volume est effacé de manière cyclique ou la valeur qui y est enregistrée est réduite cycliquement d'une valeur prédéfinie.

3. Procédé selon la revendication 1 ou 2, **caractérisé en ce que** la grandeur dérivée enregistrée dans le registre (11) est le produit du volume et de la différence de température entre la température mesurée de l'eau et la valeur de seuil de température.

4. Procédé selon la revendication 1 ou 2, **caractérisé en ce que** l'indice formé est le quotient du volume enregistré dans le registre (11) et du volume total qui s'est écoulé à travers.

5. Procédé de surveillance du potentiel de risque concernant une contamination microbienne au niveau d'un compteur débitmètre (1), selon lequel
- suivant une température de l'eau déterminée par une sonde de température (10) et en cas de dépassement d'au moins une valeur de seuil de température pour la température de l'eau, au moins le temps détecté par le compteur débitmètre (1) lorsque le compteur est à l'arrêt et/ou une grandeur qui en est dérivée sont enregistrés dans au moins un registre (12) d'un dispositif de mémorisation (5) et
- un indice pour le risque de contamination microbienne est déterminé à partir de l'au moins une grandeur enregistrée dans le registre (12) et interprété.

6. Procédé selon la revendication 5, **caractérisé en ce que** le registre (12) associé au temps est effacé lorsque la température de l'eau, pendant une opération de soutirage, devient inférieure à une valeur prédéfinie, notamment à la valeur de seuil de température.

7. Procédé selon la revendication 5 ou 6, **caractérisé en ce qu'**en présence d'une température inférieure à la valeur de seuil de température basse associée au registre (12), la valeur enregistrée dans le registre (12) est diminuée en fonction du temps.

8. Procédé selon l'une des revendications 5 à 7, **caractérisé en ce que** l'indice formé est le quotient du temps enregistré dans le registre (12) et d'un temps total.

9. Procédé selon l'une des revendications précédentes, **caractérisé en ce qu'**un signal d'alarme est délivré en cas de dépassement d'une valeur de seuil d'alarme pour l'indice.

10. Procédé selon l'une des revendications précédentes, **caractérisé en ce que** l'indice ou une information qui en est dérivée, notamment le signal d'alarme, est transmis(e) sans fil, notamment par voie radioélectrique, à un autre dispositif (8).

11. Procédé selon l'une des revendications précédentes, **caractérisé en ce que** plusieurs registres associés à une grandeur physique sont prévus pour différentes valeurs de seuil de température et/ou différents intervalles de température.

12. Procédé selon l'une des revendications précédentes, **caractérisé en ce que** lors de la détermination de la grandeur dérivée de la grandeur physique, le tracé dans le temps de la température de l'eau, notamment le différentiel du tracé de la température, est pris en compte.

13. Compteur débitmètre (1) pour l'eau, comprenant un dispositif de calcul (4) configuré pour mettre en ouvre le procédé selon l'une des revendications précédentes et un dispositif de mémorisation (5).
